(19) Europäisches Patentamt / European Patent Office / Office européen des brevets

(11) **EP 2 101 633 B1**

(12) **EUROPEAN PATENT SPECIFICATION**

(45) Date of publication and mention
of the grant of the patent:
**20.07.2011 Bulletin 2011/29**

(21) Application number: **07857307.8**

(22) Date of filing: **10.12.2007**

(51) Int Cl.:
*A61B 5/00* (2006.01)    *A61B 5/02* (2006.01)

(86) International application number:
**PCT/EP2007/063572**

(87) International publication number:
**WO 2008/071643 (19.06.2008 Gazette 2008/25)**

(54) **DEVICE FOR CONTINUOUS, NON-INVASIVE MEASUREMENT OF ARTERIAL BLOOD PRESSURE AND USES THEREOF**

VORRICHTUNG FÜR DIE KONTINUIERLICHE, NICHT-INVASIVE MESSUNG DES ARTERIELLEN BLUTDRUCKS UND IHRE VERWENDUNG

DISPOSITIFS DE MESURE CONTINUE NON INVASIVE DE LA PRESSION ARTERIELLE ET UTILISATIONS DE CEUX-CI

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **11.12.2006  AT 20432006**
**08.02.2007  US 888845 P**

(43) Date of publication of application:
**23.09.2009 Bulletin 2009/39**

(73) Proprietor: **Cnsystems Medizintechnik Gmbh**
**8020 Graz (AT)**

(72) Inventors:
• **FORTIN, Jürgen**
  **8020 Graz (AT)**
• **GRÜLLENBERGER, Rupert**
  **8020 Graz (AT)**

(74) Representative: **Babeluk, Michael**
**Patentanwalt**
**Mariahilfer Gürtel 39/17**
**1150 Wien (AT)**

(56) References cited:
WO-A-2005/037097        US-A- 5 676 140
US-A1- 2005 256 386

**Description**

**FIELD OF THE INVENTION**

The invention relates to a signal processing device, in particular a method and device for the continuous, non-invasive measuring of arterial blood pressure.

**BACKGROUND OF THE INVENTION**

The continuous monitoring of blood pressure in an artery in a non-invasive way (Continuous Non-invasive Arterial Pressure CNAP) has for many years been a topic for scientists and researchers. In 1942 R. Wagner in Munich presented a mechanical system for recording the arterial pressure in the A. radialis by means of the so-called "Vascular Unloading Technique" - the principle of the unloaded arterial wall. (Wagner R. "Methodik und Ergebnisse fort-laufender Blutdruckschreibung am Menschen", Leipzig, Georg Thieme Verlag, 1942; Wagner R. et al. "Vereinfachtes Verfahren zur fortlaufenden Aufschrift des Blutdrucks beim Menschen", Zschr. Biol. 112, 1960). The method of non-invasive determination of blood pressure presented by J. Penaz 1973 in Dresden (Digest of the 10th International Conference on Medical and Biological Engineering, 1973, Dresden) also uses the vascular unloading technique. This allows for the first time a continuous recording of intra-arterial blood pressure by means of an electro-pneumatic control loop. In this method light is shone through a finger, and via a finger cuff and a servo-mechanism pressure is applied to the finger in such a way that the originally pulsating flow detected by the transmitted light is held constant.

In principle the method is as follows. Light from at least one light source is passed through a limb or part of the human body containing an artery, such as a finger, the wrist, or the temple. The light, which is transmitted through the limb (e.g. the finger) or is reflected from a bone (e.g. wrist or temple), is registered by a suitable light detector and serves as a measure for the volume of blood in the limb or body part (plethysmographic signal s(t)), or more precisely for the blood flow in the limb, which is defined as the volume change per time. The more blood there is in the limb, the more light is absorbed and the smaller is s(t). The mean value $s_{mean}$ is subtracted from s(t) and the resulting $\Delta s(t)$ is fed into a controller. The control signal output by the controller is amplified, added to a constant set-point value SP and applied to a servo- or proportional valve, which generates pressure in a cuff placed over or on the limb or body part exposed to the light.

The control mechanism is such that $\Delta s(t)$ is kept constant over time by the applied pressure. When the heart pumps more blood into the limb during the systole and $\Delta s(t)$ decreases, the controller will increase the control signal and pressure in the cuff enclosing the limb will rise until the excess blood is pushed out of the limb and $\Delta s(t)$ assumes its former value. On the other hand, when less blood flows into the limb during diastole, because the heart is in its fill-up phase, and when therefore $\Delta s(t)$ increases, the controller will decrease the control signal and thus reduce the pressure on the finger. Again $\Delta s(t)$ is kept constant. Due to the control mechanism described ($\Delta s(t)$ and thus the arterial blood volume in the limb remain constant over time), the pressure difference between intra-arterial pressure and applied external pressure (the so called transmural pressure) is zero. Thus the applied external pressure equals the intra-arterial pressure in the limb, which therefore can be measured continuously and non-invasively by means of a manometer.

The above description of the Penaz principle assumes the control loop to operate in "closed loop" mode. The control loop may also be opened ("open loop"), i.e. with the control signal not being added to the set-point value SP. In this case the cuff pressure will not depend on $\Delta s(t)$, but is determined by SP. In this operating mode the optimum SP for the limb is found. According to Penaz this SP corresponds to the mean arterial blood pressure in the limb and is characterized by maximal pulsations of $\Delta s(t)$.

The tacit assumption is that the pulsating signal $\Delta s(t)$ obtained from the transmitted light corresponds exactly to the arterial blood flow as a function of time in the body part (usually the finger) measured. This is only the case, however, if the blood in the sensor area flows uniformly through the capillary bed and if the venous return flow is constant. The arterial-venous blood flow is quite variable, however. Changes in venous light absorption are therefore a significant source of error in the vascular unloading signal and the arterial blood pressure measured with the use of this signal.

The photoplethysmographic method according to Penaz, which is also known as "vascular unloading technique" or in some publications as "volume clamp method", has been further improved. EP 0 537 383 A1 (TNO), for instance, shows an inflatable finger cuff for non-invasive, continuous blood pressure monitoring. The inflatable cylindrical chamber of the cuff is pneumatically connected to a fluid source. An infrared light source and a detector are positioned inside the rigid cylinder on opposite sides of the finger. A valve for filling the cylinder with gas is provided. Electrical leads for the infrared light source and the detector are passed through the cylinder wall. U. S. Pat. No. 4,510,940 A (Wesseling) and U.S. Pat. No. 4,539,997A (Wesseling) show devices for the continuous, non-invasive measurement of blood pressure. A fluid-filled cuff, a light source, a light detector and an amplifier for the pressure difference are provided. U.S. Pat. No. 4,597,393 (Yamakoshi) also discloses a variant of the Penaz principle.

In WO 00/59369 A2 improvements in valve control or rather in the pressure generating system and variants of the pressure cuffs (e.g. a double cuff) for diverse limbs or body parts are shown. WO 04/086963 A2 contains a description of how the double cuff can be used to measure blood pressure according to the Penaz principle in one cuff, while the other cuff is used for optimised control of the set-point SP. WO 05/037097 A1 describes an improved control system for the vascular unloading technique, where interior control loops provide quasi optimised conditions for succeeding exterior control loops.

While the publications cited above represent improvements of the vascular unloading technique they still tacitly assume that the pulsatile component $\Delta s(t)$ of the plethysmographic signal $s(t)$ corresponds to the arterial signal component, or rather the arterial blood flow.

From pulsoximetry (an optical method for the non-invasive determination of oxygen saturation) it is known that motion artefacts corrupting the arterial signal $a(t)$, can be eliminated by suitable measures. In US Patent Nos 4,653,498 A, 5,025791A, 4,802,486A, 5,078,136A, 5,337,744A, and 6,845,256A methods are cited which may be employed to remove such motion artefacts from the measured signals. Separation of the arterial signal $a(t)$ from the venous signal $v(t)$, however, cannot be based on these methods, and they are not an object of the present invention.

In US 2005/0256386 a device and a method for venous pulse oximetry is described. The method applies pressure pulsations on a first site of the body whereas on a different second site an oximeter device is applied. The method extracts venous oxygen saturation by applying such pulsations with a frequency distinguishable from heart rate.

In the patents and patent applications US 5,769,785A, US 6,036,642A, US 6,157,850A, US 6,206,830A, US 6,263,222A, WO 92/15955, EP 0 574 509 B1, DE 692 29 994, WO 96/12435 A2 novel methods of signal analysis are described, which are used to eliminate from two or more plethysmographic signals the unwanted signals, such that a favored signal for the measurement of oxygen saturation via pulsoximetry remains. In these publications methods for signal analysis such as "Linear Relationship", "Adaptive Filter", "Adaptive Signal Processor", "Adaptive Noise Canceler", "Self Optimizing Filter" and "Kalman Filter" are described among others. These signal analysis methods are employed not only in electronics but also in medicine for medical or physiological signals. (A.F.M. Smith and M. West: "Monitoring Renal Transplants: An Application for the Multiprocess Kalman Filter", Biometrics 39 (1983) p. 867-878; K. Gordon: "The Multi State Kalman Filter in Medical Monitoring", Computer Methods and Programs in Biomedicine 23 (1986), p. 147-154).

## SUMMARY OF THE INVENTION

The present invention provides improved signal processing devices that provide a clear separation between favored and supplementing signals of one first and at least one second, time-varying quantity, and in particular a device and a method for the continuous, non-invasive measurement of arterial blood pressure, by which a clear separation can be achieved between the (favored) arterial signal $a(t)$ and the (supplementing) venous signal $v(t)$ of blood volume or blood flow, as defined in claims 1 and 5.

an embodiment A provides a signal processing device comprising:

(a) at least one detector for generating at least one measurement signal from at least one measurement radiation, wherein the measurement radiation propagates along a propagation medium starting from at least one radiation source;
(b) an air pressure generator, one or more valves, a manometer and a cuff for applying a pressure on the propagation medium;
(c) a reference signal generator that accepts the signals generated by the detector and the pressure generated by the pressure generator to compute a reference signal; and
(d) a filter receiving the reference signal as an input, wherein the filter essentially separates a supplementing signal and a favored signal from the signals generated by the detector,

wherein the favored signal is a measure of the physiological characteristics.

In aspect according to embodiment A, each of the measurement radiation of (a) is of different wavelength. In one other aspect, the measurement radiation of (a) propagates wholly or partially along a propagation path situated in the propagation medium. The the propagation medium can be a human body part. In still another aspect, the pressure of (b) is a time-variablc pressure.

Another embodiment B provides a device for measuring one or more physiological characteristics, the device comprising

(a) at least one radiation source for generating at least one measurement radiation, wherein the measurement radiation propagates through a body part;
(b) at least one detector for generating at least one measurement signal from the measurement radiation;
(c) an air pressure generator, one or more valves, a manometer, and a cuff for applying a pressure to the body part;
(d) a reference signal generator, which computes a reference signal from the signal generated by the detector and the pressure signal from the pressure generator; and

(e) a filter receiving the reference signal, wherein the filter essentially separates a supplementing signal and a favored signal from the signals measured by the detector, wherein the favored signal is a measure of the physiological characteristics.

In one aspect according to embodiment B, each of the measurement radiation of (a) is of different wavelength, or mutually differing wavelengths. In another aspect, the measurement radiation of (a) propagates wholly or partially along a propagation path situated in the body part. In one other aspect, the pressure of (c) is a time-variable pressure. In still another aspect, the physiological characteristics comprise blood characteristics, arterial and venous characteristics, blood pressure characteristics, arterial oxygen saturation, or venous oxygen saturation.

One other embodiment C provides a device comprising:

(a) at least one detector providing a first measurement signal $s_1(t)$ from a measurement radiation of defined wavelength, which propagates along a propagation path starting from a first radiation source, and at least one other measurement signal $s_N(t)$ from another measurement radiation of different wave-length, which propagates wholly or partially along the propagation path starting from at least one other radiation source, wherein at least a portion of the propagation path is situated in a propagation medium, wherein the first signal $s_1(t)$ comprises a favored signal $a_1(t)$ and a supplementing signal $v_1(t)$ and the at least one other signal $s_N(t)$ comprises a favored signal $a_{N(t)}$ and a supplementing signal $v_N(t)$, wherein the signals $a_1(t)$ to $a_N(t)$ result from a first, time-variable quantity $a(t)$ in the propagating medium and the signals $v_1(t)$ to $v_N(t)$ result from a second, time-variable quantity $v(t)$ in the propagation medium;

(b) an air pressure generator, one or more valves, a manometer and a cuff for applying time-variable pressure on the propagation medium, with a pressure signal $p(t)$ being a function of the first, time-variable quantity $a(t)$ of the propagation medium or a function of one or more signals $s_1(t)$ to $s_N(t)$ measured by the detector;

(c) a reference signal generator, which accepts the signals $s_1(t)$ to $s_N(t)$ measured by the detector and the pressure signal $p(t)$ as inputs and computes from these inputs a reference signal $\Delta n'(t)$, which is a function of the second, time-variable quantity $v(t)$ or of the supplementing signals $v_1(t)$ to $v_N(t)$; and

(d) a filter receiving the reference signal $\Delta n'(t)$ as an input, wherein the frequency properties of the filter essentially correlate with the reference signal $\Delta n'(t)$, and wherein the filter essentially separates from at least one of the signals $s_1(t)$ to $s_N(t)$ measured by the detector the supplementing signal $v_1(t)$ to $v_N(t)$ from the favored signal $a_1(t)$ to $a_N(t)$.

A preferred embodiment D comprises a device for the continuous, non-invasive measurement of arterial blood pressure, the device comprising:

(a) a first radiation source and at least one other radiation source for generating a first and at least one other measurement radiation of defined, mutually differing wavelengths;

(b) at least one detector for generating a first measurement signal $s_1(t)$ from the first measurement radiation and at least one other measurement signal $s_N(t)$ from the at least one other measurement radiation of different wavelength, wherein the measurement radiations propagate wholly or partially along a propagation path and wherein at least a portion of this propagation path is located in a body part traversed by arterial and venous blood flows, and wherein the first signal $s_1(f)$ has a first arterial signal component $a_1(t)$ and a first venous signal component $v_1(t)$ and wherein the at least one other signal $s_N(t)$ has at least one other arterial signal component $a_N(t)$ and at least one other venous signal component $v_N(t)$, and wherein arterial signal components $a_1(t)$ to $a_N(t)$ result from a time-varying arterial blood flow $a(t)$ in the body part, and the venous signal components $v_1(t)$ to $v_N(t)$ result from a time-varying venous blood flow $v(t)$ in the body part;

(c) an air pressure generator, one or more valves, a manometer and a cuff for applying a time-varying pressure to the body part, wherein a pressure signal $p(t)$ corresponding to an arterial blood pressure, is a function of the arterial blood flow $a(t)$ in the body part or a function of one or more of the signals $s_1(t)$ to $s_N(t)$ measured by the detector;

(d) a reference signal generator, which has as inputs the signals $s_1(t)$ to $s_N(t)$ measured by the detector and the pressure signal $p(t)$, and which computes from these inputs a reference signal $\Delta n'(t)$, which is a function of the venous blood flow $v(t)$ or of the venous signal components $v_1(t)$ to $v_N(t)$; and

(e) a filter receiving the reference signal $\Delta n'(t)$ as an input, where the frequency properties of the filter essentially correlate with the reference signal $\Delta n'(t)$, and wherein the filter essentially separates from at least in one of the signals $s_1(t)$ to $s_N(t)$ measured by the detector the venous signal component $v_1(t)$ to $v_N(t)$ from the arterial signal component $a_1(t)$ to $a_N(t)$, wherein the arterial signal component is proportional to the arterial blood flow $a(t)$.

The device according to the invention achieves a clear separation between the arterial (favored) signal component (e.g. $a_1(t)$) and the venous (supplementing) signal component (e.g. $v_1(t)$) of the measurement signal. Thus it is possible to use exclusively the signal component of the arterial blood a(t) as the input variable for the vascular unloading technique.

The filtered-out venous signal component v(t) may for instance be used to correct another disadvantage implicit in the conventional version of the vascular unloading technique. By the counter-pressure on the body part measured the venous out-flow from the sensor area is impeded and the finger turns blue - local cyanosis occurs. By monitoring the venous signal component and the venous oxygen saturation the system may be switched off or switched over to another sensor before the measuring situation is turning unpleasant for the patient. Due to the separation of arterial and venous signals the oxygen saturation of arterial as well as venous blood may be measured and displayed.

Separating the favored from the supplementing signal as such is known from modem communication engineering and electronics, but in the present context it is necessary to know further characteristic attributes of the two signals. The invention makes use of the fact that arterial blood has an absorption coefficient differing from that of venous blood at a certain wavelength of light. Furthermore the characteristic feature of the vascular unloading technique must be considered in the separation process, i.e. that the signal obtained from the passing or reflected light is minimized by the counter- pressure applied.

One other embodiment E, provides a pulse oximeter comprising

(a) at least one radiation source for generating at least one measurement radiation, wherein the measurement radiation propagates through a body part;
(b) at least one detector for generating at least one measurement signal from the measurement radiation;
(c) an air pressure generator, one or more valves, a manometer, and a cuff for applying a time-varying pressure to the body part;
(d) a reference signal generator, which computes a reference signal from the signal generated by the detector and the pressure signal from the pressure generator; and
(e) a filter receiving the reference signal, wherein the filter essentially separates a supplementing signal and a favored signal from the signals measured by the detector, wherein the favored signal is a measure of the physiological characteristics.

One other embodiment F provides a pulse a method for measuring one or more physiological characteristics, the device comprises

(a) providing a first and at least one other measurement radiation;
(b) detecting a first measurement signal from the first measurement radiation and at least one other measurement signal from the at least one other measurement radiation of different wavelength, where the two measurement radiations propagate wholly or partially along the same propagation path in a body part;
(c) applying a pressure to the body part;
(d) computing a reference signal from the first and the at least one measurement signals of (b) and the pressure of (c); and
(e) separating a supplementing signal component and a favored signal component from the measurment signals of (b) by using a filter that receives a reference signal as an input, wherein the reference signal is computed from the measurement signal of (b) and the pressure signal of (c),

wherein the favored signal component is a measure of the physiological characteristics.

In one aspect according to embodiment F, each of the measurement radiation of (a) is of different wavelength or mutually differing wavelengths. In another aspect, the measurement radiation of (a) propagates wholly or partially along a propagation path situated in the body part. In one other aspect, the pressure of (c) is a time-variable pressure. In still another aspect, the physiological characteristics comprise blood characteristics, blood characteristics, arterial and venous characteristics, blood pressure characteristics, arterial oxygen saturation, or venous oxygen saturation. The invention also provides a method for a continuous, non-invasive measurement of arterial blood pressure in a body part traversed by arterial and venous blood flows comprising:

(a) providing a first and at least one other measurement radiation of defined, mutually differing wavelengths;
(b) detecting a first measurement signal $s_1(t)$ from the first measurement radiation and at least one other measurement signal $s_N(t)$ from the at least one other measurement radiation of different wavelength, where the two measurement radiations propagate wholly or partially along the same propagation path and wherein part of this propagation path is located in the body part in which arterial and venous blood flows, and wherein the first signal $s_1(t)$ has a first favored signal component $a_1(t)$ and a first supplementing signal component $v_1(t)$, and wherein the at least one other signal $s_N(t)$ has a favored signal component $a_N(t)$ and a supplementing signal component $v_N(t)$, and wherein the first and all other favored signal components $a_1(t)$ to $a_N(t)$ result from a time-varying arterial blood flow a(t) in the body part and the first and all other supplementing signal components $v_1(t)$ to $v_N(t)$ result from a time-varying venous blood flow $v(t)$ in the body part;

(c) applying a time-varying pressure to the body part, wherein a pressure signal $p(t)$ corresponding to the arterial blood pressure is a function of the arterial blood flow $a(t)$ in the body part or a function of one or more of the signals $s_1(t)$ to $s_N(t)$;

(d) computing a reference signal $\Delta n'(t)$ from the signals $s_1(t)$ to $s_N(t)$ and the pressure signal $p(t)$, which is a function of venous blood flow $v(t)$ or of the supplementing signal components $v_1(t)$ to $v_N(t)$; and

(e) separating the supplementing signal component $v_1(t)$ to $v_N(t)$ from the favored signal component $a_1(t)$ to $a_N(t)$ of the signals $s_1(t)$ to $s_N(t)$ measured by a detector by means of a filter receiving the reference signal $\Delta n'(t)$ as an input, wherein the frequency properties of the filter essentially correlates with the reference signal $\Delta n'(t)$, and wherein the favored signal component $a_1(t)$ to $a_N(t)$ is proportional to the arterial blood flow $a(t)$.

## BRIEF DESCRIPTION OF THE DRAWINGS

Fig. 1 shows a device according to the invention for the continuous, non-invasive measurement of arterial blood pressure.

Fig. 2 shows the device of Fig. 1 with a first variant of the filter.

Fig. 3 shows the device of fig. 1 with a second variant of the filter.

Fig. 4 shows the relationship between optical density ratio r and oxygen saturation SpO2 in the form of a calibration curve.

Figs. 5a to 5c show variants of the output-power diagrams of the filters.

Figs. 6a to 6c show further variants of the output-power diagrams of the filters.

## DESCRIPTION OF THE INVENTION

The invention relates to methods and devices for continuous, non-invasive measurement of arterial blood pressure.

The term "physiological characteristics" comprises any type of physiological parameter. For example, physiological characteristics include, but are not limited to blood characteristics, arterial blood flow characteristics, venous blood flow characteristics, blood pressure characteristics, arterial oxygen saturation, or venous oxygen saturation. Physiological characteristics also include blood glucose concentration, blood $CO_2$ concentration, arterial blood glucose concentration, arterial blood $CO_2$ concentration, venous blood glucose concentration, and venous blood $CO_2$ concentration.

The term "measurement radiation" or "radiation" comprises any type of energy form such as waves or moving subatomic particles. Radiation includes, but not limited to, visible light, electromagnetic waves, sound, ultrasound, and ionizing or non-ionizing radiation.

The term "measurement signal" is the radiation detected by a detector after passage through a propagation medium.

The term "propagation medium" comprises any part of the human or animal body. For example, a propagation medium is a portion of a finger, ear, or arm.

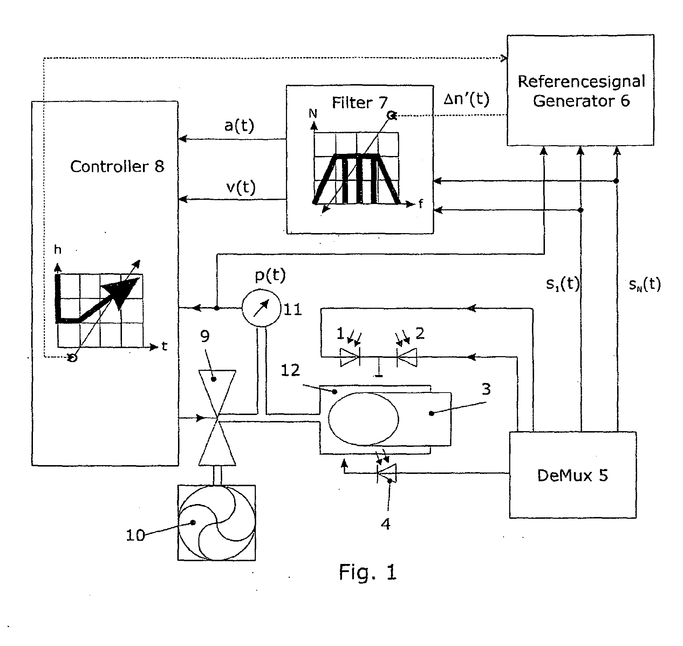

In one embodiment, the invention provides methods for continuous, non-invasive measurement of arterial blood pressure comprising a device as illustrated in Fig. 1. The device comprises(a) a first radiation source (1) and at least one other radiation source (2), which provides a first and at least one other measurement radiation of defined mutually differing wavelengths; (b) at least one detector (4), which provides a first measurement signal $s_1(t)$ from the first measurement radiation and at least one other measurement signal $s_N(t)$ from another measurement radiation of different wavelength, the two measurement radiations propagating wholly or partially along the same propagation path, part of the propagation path being located in a body part (3) with arterial and venous blood flow, where the first signal $s_1(t)$ consists of a favored signal $a_1(t)$ and a supplementing signal $v_1(t)$ and the at least one further signal $s_N(t)$ consists of a favored signal $a_N(t)$ and a supplementing signal $v_N(t)$, with the first and further favored signals $a_1(t)$ to $a_N(t)$ being the result of the time-variable arterial blood flow $a(t)$ in the body part (3), and the first and further supplementing signals $v_1(t)$ to $v_N(t)$ being the result of the time-variable venous blood flow $v(t)$ in the body part (3); (c) an air pressure generator, one or more valves, a manometer and a cuff (9, 10, 11, 12) for applying time-variable pressure on the body part, wherein a pressure signal $p(t)$, which corresponds to the arterial blood pressure, being a function of the arterial blood flow $a(t)$ in the body part or a function of one or more signals $s_1(t)$ to $s_N(t)$; (d) a reference signal generator (6), which accepts the signals $s_1(tt)$ to $s_N(t)$ and the pressure signal $p(t)$ as inputs and computes from the inputs a reference signal $\Delta n'(t)$; and (e) a filter (7), which receives, the reference signal $\Delta n'(t)$ as an input, wherein the filter essentially separates from at least in one of the signals $s_1(t)$ to $s_N(t)$ the supplementing signal $v_1(t)$ to $v_N(t)$ from the favored signal $a_1(t)$ to $a_N(t)$.

In one embodiment of the method, the frequency properties of the filter are adaptively modified during signal analysis by means of the reference signal. In another embodiment, from the frequency properties obtained by measuring the blood pressure, the arterial oxygen saturation aSp02 and/or the venous oxygen saturation vSp02, are derived and displayed. In yet another embodiment, the red light is used as the first measurement radiation and infrared light is used as the second measurement radiation. In still another embodiment, the red light is of wavelength 660 nm and the infrared light is of wavelength 940 nm.

The essential difference between the present invention and the state of the art as regards oxygen saturation, lies in the fact that the element for separating the arterial (favored) signal component from the venous (supplementing) signal component (e.g. a filter or other suitable means for signal analysis) is located in a control loop. This control system applies energy, i.e. pressure on the body part measured, which pressure corresponds to the arterial blood pressure. This pressure changes the measured plethysmographic signals of all wavelengths at the said body part and minimizes the arterial signal component a(t). Ideally, this signal approaches zero.

The applied pressure furthermore depends directly on the favored signal. This arterial (favored) signal a(t) influences the measuring of the desired signal - the arterial blood pressure -, whose equivalent is applied to the body part as counter-pressure. The plethysmographic signals necessary for generating and controlling this pressure - i.e. the signals measured by the light sensors - act back on themselves via the control loop. This will necessarily also influence the working of the signal analysis procedures, since the applied pressure modulates also the venous (supplementing) signal v(t), which will thus be no longer independent of the arterial signal a(t). The fact that a(t) and v(t) no longer are independent signals must be taken into account in a suitable way. This requires yet another degree of freedom in the control loop, which might for instance be achieved by utilizing the fact that the arterial signal a(t) is minimized if the control loop is in the optimal state, and ideally will even tend to zero.

The filter used to separate the arterial (favored) signal a(t) from the venous (supplementing) signal v(t) needs a reference signal n(t) to determine the filter properties, which will be described in more detail later on. In EP 0 574 509 B1 this reference signal is obtained from light signals and their correlations. For the present invention, however, it is indispensable that the pressure p(t) applied at the body part measured be considered in the building of the reference signal n(t). This constitutes a further essential difference between the invention and the state of the art.

The pressure applied to the body part will also lead to physiological changes. The arterial blood supply of the body part will always be ensured since the artery is not clamped by the externally applied pressure, but only the diameter of the artery and thus the blood volume measured via the plethysmographic signal, is kept constant. Due to this fact the vascular unloading technique is also termed "volume clamp method". The situation is different for the capillary bed and the venous blood flow, which will be impeded by the pressure applied, until pressure in the system of venous blood vessels is equal or greater than the applied pressure. Only in this case venous back-flow will set in. The circumstance mentioned above, i.e. that the venous signal is modulated by the pressure, which in turn is generated by the arterial signal, thus is not only a computational fact, but occurs in reality. Due to the impeded venous back-flow the respective body part in most patients assumes a blue colour (cyanosis), which however is harmless, since the supply with oxygen-rich arterial blood will always be ensured. Increased pressure in the capillary bed and in the veins has as a necessary consequence that more erythrocytes release their oxygen molecules, since they remain longer at the site of exchange, and thus oxygen saturation of the venous blood will decrease in the area of measurement. This circumstance as such is harmless for the patient but must be considered when oxygen saturation is to be measured; it can furthermore be utilized to implement a safety measure in the system. If the arterial blood supply is interrupted due to a malfunction, this can be detected by monitoring oxygen saturation, and the system will automatically close down or resume measuring at another part of the body. This safety monitoring function is another advantage of the present invention.

Determination of the oxygen saturation of arterial and venous blood by means of the same sensor used for measuring arterial blood pressure is a further advantageous development of the present invention. Conventional pulsoximetry, which determines the ratio of optical density r and, consequently, the oxygen saturation Sp02 from the two pulsatile plethysmographic signals, will not work here. The fact that the arterial signal as such but also the venous signal via the applied pressure contribute to the pulsatile signal components, would corrupt the determination of the optical density r. A filter or another suitable signal analysis procedure for separating arterial from venous blood must be provided for oxygen saturation measurement. Furthermore, it must be taken into account that the arterial (favored) signal is minimized by the control loop. The filter, which is already present in the control loop for measuring arterial blood pressure, will take care of these points, thus making oxygen saturation measurement an advantageous side product of the present invention.

JP 06-063024 A2 (Igarashi et al.) and JP 02-305555 A (Yamakoshi) describes an instrument for the simultaneous determination of blood pressure and oxygen saturation Sp02 in one sensor. The Penaz method is simply extended in that instance by providing a second light source with different wavelength. While the pulsatile components of one light signal are used for the vascular unloading technique of blood pressure measurement, the oxygen saturation is found from the ratio of the two pulsating components. No filter or other suitable procedure of signal analysis is provided to separate arterial blood components from venous blood components in the two signals of differing wavelengths. Furthermore no measures are proposed which would take into account the changes in the plethysmographic signals due to the pressure applied, as described above. Corruption of the measured values is to be expected due to the changed venous back-flow, which is modulated via the control loop by the arterial signal. To put it simply; the Sp02-value will be significantly corrupted by the counter-pressure applied and the resulting venous congestion at the measuring site. The existing oxygen saturation is underestimated.

U.S. Pat. No. 5,485,838 A (Ukawa et al.) is not a device for continuous blood pressure measurement and does not have reference signal generators. Further, the filters correspond to different criterias than in the present application.

U.S. Pat. No. 5,111,817 A (Clark et al.) also describes a system and a method for the simultaneous determination of blood pressure and oxygen saturation. Once more a cuff is provided with a second light source of different wavelength. A control loop, which would be necessary for continuous determination of blood pressure by the Penaz or vascular unloading method, is lacking, however. Blood pressure is determined by obtaining the plethysmographic signals at certain defined constant pressures in the cuff. From the pressure-volume ratios a so-called Hardy model is computed, which will then be responsible for determining the blood pressure from the plethysmographic signals. The system is further marked not only by the absence of the control loop but also by the lack of a filter for separating arterial and venous signal components.

U.S. Patent No. 4,927,264 A (Shiga et al.) also discloses a cuff and a second light source with different wavelength in the same sensor. In that case the object is a method and device for measuring venous oxygen saturation, a control loop and a filter for separating arterial from venous signal components again being absent.

It is to be noted that all circuits mentioned in the context of the present invention can be implemented both as hardware, e.g. as an electronic printed circuit, and as software, e.g. as a program in a computer or a digital signal processor DSP.

The invention will now be described in more detail with reference to the enclosed, partly schematic drawings.

Fig. 1 shows a general control loop of the device of the invention for continuous, non-invasive measurement of arterial blood pressure, which comprises a filter 7 for separating the signal components. A radiation source or light source I and at least one further radiation or light source 2 of a different wavelength transmit light through a body part 3 containing an artery. This is preferably done with light emitting diodes (LEDs) or laser diodes emitting red or infrared light. A suitable body part is for instance a finger with its A. digitalis or the temple with the A. temporalis, where light is reflected by the temporal bone. The body part 3 absorbs light in differing degrees depending on arterial or venous blood flow. The absorption at differing wavelengths also depends on the oxygen content of the blood. It is well known that oxygen-rich blood is red, while blood deficient in oxygen is bluish. The absorbed radiation of at least two different wavelengths is measured at a suitable site by one or more detectors 4 (for instance photodiodes). In order to distinguish between the signals of the different wavelengths a demultiplexer 5 is preferably provided. This device also controls the switching-on of the light sources 1 and 2, and thus generates two or more signals (e.g. $s_1(t)$, $s_2(t)$ to $s_N(t)$), which correspond to the absorption of radiation at the individual wavelengths. The two signals also serve as a measure of the blood volume which is present in the body part 3 at each moment, or as a measure of the blood flow, which is defined as volume change $\Delta V$ per unit of time.

The at least two signals $s_1(t)$ and $s_2(t)$ to $s_N(f)$ are now passed to a reference signal generator 6, which generates from the signals $s_1(t)$, $s_2(t)$ to $s_N(f)$ together with the pressure signal $p(t)$, which will be described later on, a signal $\Delta n'(t)$ having the same frequency properties as one of the signals $a(t)$ or $v(t)$. This reference signal is used by the following filter 7 to adapt itself according to the prevailing frequency properties. Thus the filter 7 can distinguish between arterial and venous blood volume or flow $a(t)$ and $v(t)$ in the body part 3. The two signals $a(t)$ and $v(t)$ are fed to a controller 8, which, by means of an assembly comprising one or more valves 9, an air pressure generator or a pump 10 and a cuff 12, will generate a pressure $p(t)$ measured by a manometer 11. This pressure $p(t)$ will act in the cuff 12 covering the body part 3 to be monitored. The control mechanism of the controller 8 is such that the arterial signal or the arterial blood flow $a(t)$ is kept constant over a period of time by means of the pressure $p(t)$. The characteristic of the controller 8 will also influence retroactively the characteristic of the reference signal generator 6 and hence the filter 7.

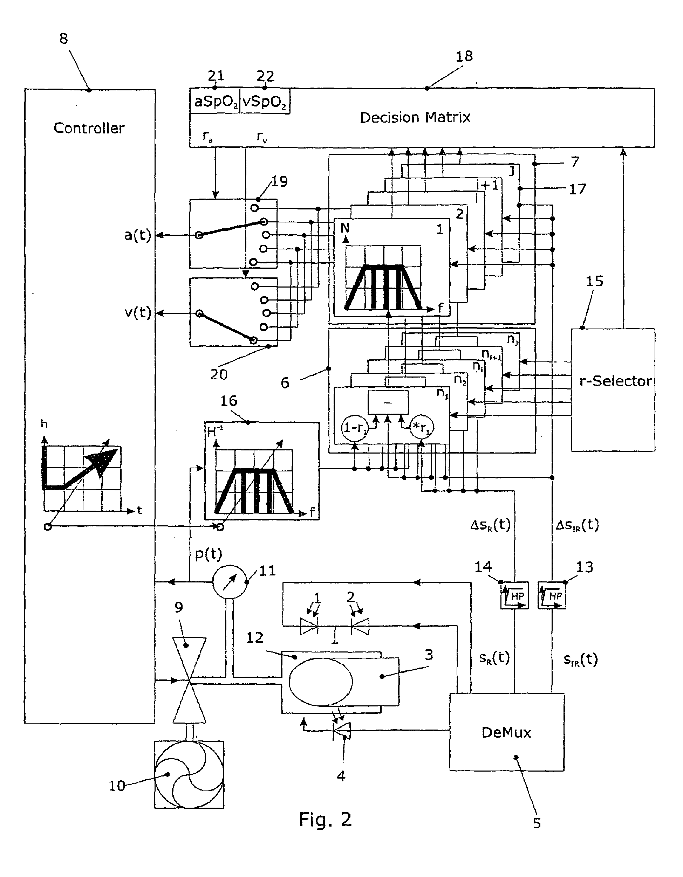

Fig. 2 shows a possible variant of the filter and the diverse influences on the determination of the filter characteristics N or the controller transfer function h. While two or more signals of differing wavelengths may be used for the present invention, it is practical to use one signal of red light and one signal of infrared light. In the following the designation $s_1(t)$ and $s_2(t)$ to $s_N(t)$ for the signals will be replaced by $s_R(t)$ and $s_{IR}(t)$ for better understanding.

A so-called "bi-color LED" could for instance be used, which can be switched with high frequency between a first wavelength of e.g. 600 nm and a second wavelength of e.g. 940 nm. The two light sources I and 2 are aligned with the detector 4 along a single optical axis in this case, resulting in coinciding propagation paths of the two measuring radiations, and thus improving the measurement result.

In fig. 2 the mean values of the signals $s_R(t)$ and $s_{IR}(t)$ output by the demultiplexer 5 are suppressed at first, which can be achieved by two high-pass filters 13 and 14, for instance. From the two signals $\Delta s_R(T)$ and $\Delta s_{IR}(t)$ the reference signal generator 6 derives J different reference signals $n_1$ to $n_J$. The necessary r-values are generated by the r-selector 15. A further filter 16, which has a characteristic inverse to that of the controller 8, generates a filtered pressure signal, which is also required for the generation of the reference signals. From the J reference signals J filter characteristics for a filter matrix 17 are derived. Thus J different filters are produced, which can be used to filter the signals $s_R(t)$ and $s_{IR}(t)$. A decision matrix 18 selects from these J filters in the filter matrix 17 the ones suitable for generating a(t) and v(t), by means of the selector switches 19 and 20. The selected filters correspond to the r-values matching the arterial oxygen saturation aSp02 or $r_a$ and the venous oxygen saturation vSpO2 or $r_v$. In this way aSp02 and vSpO2 are determined and can be displayed by the displays 21 and 22.

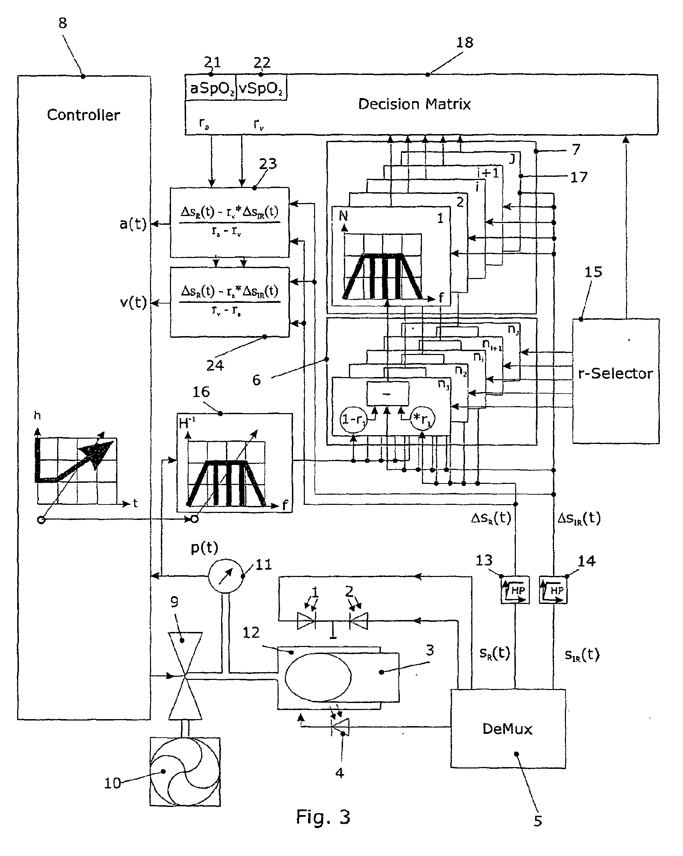

Fig. 3 shows a further variant of the filter and the diverse influences on the determination of the filter characteristics N or the controller transfer function h as regards the time-optimised determination of a(t) and v(t). In this case, instead of obtaining a(t) and v(t) with correctly selected filters having characteristics indicated by $r_a$ and $r_v$, the circumstance is utilised that the two signals a(t) and v(t) correspond to certain formulae, which will be described below. In this variant the selector switches 19 and 20 are replaced by the computation units 23 and 24. These units compute a(t) and v(t) from the given values $r_a$ and $r_v$. The r-values $r_a$ and $r_v$ can be obtained from the filter matrix 17 or the r-selector 15 without time constraints, whereas the computation units can compute a(t) and v(t) in real time.

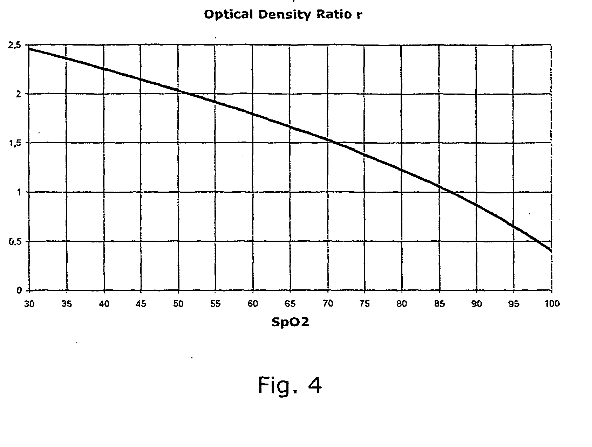

Fig. 4 shows a typical calibration curve relating optical density ratio r and oxygen saturation Sp02.

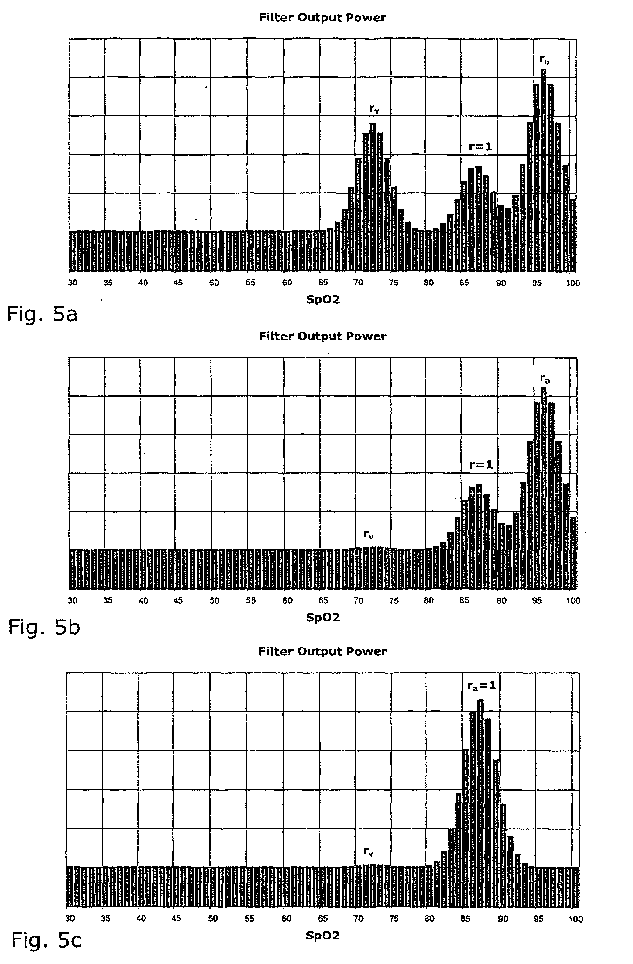

Figs. 5a to 5c show diverse possibilities for output power diagrams. Fig. 5a shows typical output power of the J filters, for arterial oxygen saturation aSp02 = 96% (era = 0.612) and venous oxygen saturation vSp02 = 72% ($r_v$ = 1.476). At r = 1 (SpO2 = 86.7%) a local peak of output power occurs due to the feedback of pressure on the body part 3, which acts on the signals $s_R(t)$ and $s_{IR}(t)$ obtained by LEDs 1 and 2. The decision matrix 18 can distinguish precisely between aSpO2 ($r_a$) and vSpO2 ($r_v$).

Fig. 5b shows filter behaviour when venous blood flow is small or only influenced by the pressure p(t), which is the case at r = 1. There is very little variable venous blood flow caused for instance by movement of the body part 3. But arterial blood flow at the site aSp02 = 96% ($r_a$ = 0.612) and the feed-back peak can be clearly seen. The decision matrix 18 recognizes that no corrupting influence due to venous blood flow is present and is able to compute a(t) directly from one of the two unfiltered signals $s_R(t)$ and $s_{IR}(t)$. Only aSpO2 can be displayed, however, which is usually sufficient for the user.

Fig. 5c shows the same kind of behaviour - here too the influence of venous blood flow on output power is small. In this instance oxygen saturation aSp02 = 87% ($r_a$ = 0.989) and thus the output power for $r_a$ is superimposed on that for r = 1. For the decision matrix 18 this signifies that aSp02 = 87% is displayed and that no corrupting influence due to venous blood flow is present (same as in fig. 5b).

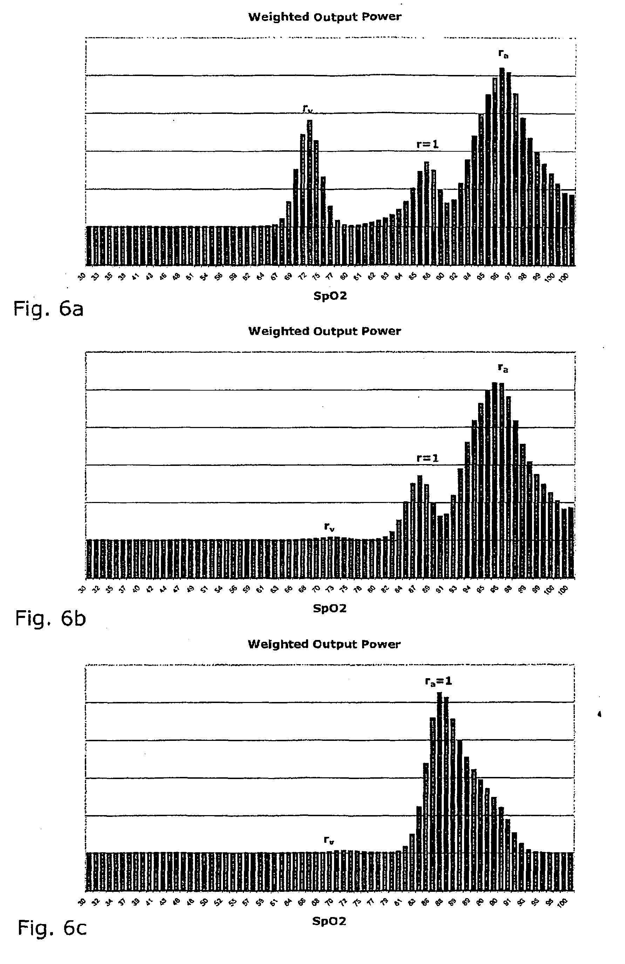

Figs. 6a to 6c show diverse possibilities of output power diagrams for weighted distances between the different r-values. The same phenomena as in figs. 5a to 5c can be observed, although the filters are better resolved at the relevant sites with high output power, thus permitting more accurate measurement of r or Sp02. It should be noted that the x-axis in these diagrams does not carry an equidistant scale, but that resolution of Sp02 changes with the amount of output power.

Control mechanism of the conventional vascular unloading technique

As described initially it is assumed in the vascular unloading technique that the arterial component of the volume signal or of the so-called plethysmographic measurement signal s(t) corresponds to the pulsatile component $\Delta s(t)$ - the constant component $s_0$ thus corresponds to the mean arterial volume, the venous back flow, the capillary component, and those portions of the light signal that are due to tissue properties. The pulsatile component is now used to control the counter-pressure p(t), the constant component of the volume signal, i.e. the mean value $s_{mem}$ being first determined and subsequently subtracted.

Assumption of the vascular unloading technique

$$s(t) = \Delta s(t) + s_0$$

with $\Delta s(t)$ supposed to be the arterial blood component a(t).

Behaviour of the controller

$$p(t)=SP + h(s(t)-s_{mean})=SP+h(\Delta s(t)+s_0-s_{mean})=SP+h(\Delta s(t)),$$

if $s_0 = s_{mean}$ and SP corresponds to mean blood pressure $p_0$ = SP.

The pressure p(t) now acts in the cuff and changes s(t), or to be more precise, $\Delta s(t)$. The control condition

states that $\Delta s(t) => 0$ and thus that the pulsatile (=arterial) component is eliminated from the volume signal s(t).

$$s(t) = \Delta s(t) + s_0 - g(p(t))$$

where g describes the relationship between cuff pressure and finger. Ideally $\Delta s(t) = g(p(t))$, or rather

$$p(t) = g^{-1}(\Delta s(t) + s0) = SP + h(\Delta s(t))$$

or

$$p(t) - p0 = g^{-1}(\Delta s(t)) = h(\Delta s(t))$$
.

and thus in the ideal case $g^{-1} = h$.

This however is theoretically only the case if no phase delay occurs and if the amplification of the controller h can become infinite. In reality phase delays occur and the amplification cannot approach infinity. Quite the opposite is the case; a control deviation i.e. a minimized but not eliminated arterial volume signal $\Delta s(t)$ must always exist, failing which no correct pressure signal p(t) can be obtained. This is important as regards the control mechanism of the present invention as described below.

Control mechanism of the present invention

The assumption of the vascular unloading technique that only arterial blood is responsible for the pulsatile component of the plethysmographic measurement signal s(t) is wrong. Capillary blood as well as venous blood can be pulsatile, especially if the patient moves the body part measured or if oxygen saturation of the blood is low. Therefore

$$s(t) = a(t) + v(t) + s_0$$

where a(t) is the arterial blood flow, v(t) designates the capillary and venous blood flow and $s_0$ subsumes all other constant components which cannot be separated (mean arterial volume, constant venous back-flow, tissue absorption). If at least two or more light frequencies are used for measurement, ideally red and infrared light, there results:

$$s_R(t) = a_R(t) + v_R(t) + s_{R0} \qquad \text{red light measured signal}$$

$$s_{IR}(t) = a_{IR}(t) + v_{IR}(t) + s_{IR0} \qquad \text{infrared light measured signal}$$

For different wavelengths of the light different absorption coefficients corresponding optical densities will exist for the arterial and the venous signal component, such that one may write:

$$a_R(t) = \dot{r}_a * a_{IR}(t) = r_a * a(t)$$

$$v_R(t) = r_v * v_{IR}(t) = r_v * v(t)$$

and thus:

$$s_{IR}(t) = a(t) + v(t) + s_{R0}$$

$$s_R(t) = r_a*a(t) + r_v*v(t) + s_{IR0}$$

$r_a$ and $r_v$ designate the optical density ratio r of arterial and venous blood. By means of empirically determined calibration curves the oxygen saturation Sp02 of arterial blood may be found from $r_a$, the oxygen saturation of venous blood from $r_v$. If both ratios are known, the filter to be described in more detail below can resolve the infrared light signal $s_{IR}(t)$ and the red light signal $s_R(t)$ into an arterial signal component $a(t)$ and a venous component $v(t)$.

First the constant part is eliminated from both signals, retaining only the pulsatile signal components:

$$\Delta s_{IR}(t) = a(t) + v(t)$$

$$\Delta s_R(t) = r_a*a(t) + r_v*v(t)$$

One may write:

$$\Delta s_R(t) = r_a*(\Delta s_{IR}(t) - v(t)) + r_v*v(t)$$

$$\Delta s_R(t) - r_a*\Delta s_{IR}(t) = r_v*v(t) - r_a*v(t)$$

And thus:

$$v(t) = \frac{\Delta s_R(t) - r_a \cdot \Delta s_{IR}(t)}{r_v - r_a}$$

$$a(t) = \Delta s_{IR}(t) - \frac{\Delta s_R(t) - r_a \cdot \Delta s_{IR}(t)}{r_v - r_a}$$

or

$$a(t) = \frac{\Delta s_R(t) - r_v \cdot \Delta s_{IR}(t)}{r_a - r_v}$$

The arterial signal a(t) is now used for controlling the vascular unloading condition, i.e. it is the input signal for the controller. It is of no importance whether the controller is of the single-stage type described by Penaz and all the other groups, or a multi-stage controller as in WO 00/59369 A2 (Fortin et al.) is employed. The controller is designed such that the input signal a(t) is reduced to zero by increasing or decreasing the output pressure in the cuff. In the case of an optimal controller, a(t) = 0 and p(t), which is generated by the controller, corresponds to the arterial pressure in the finger $p_a(t)$.

$$p(t) = SP + h(a(t))$$

Pressure in the cuff also acts on the measured plethysmographic signals $s_R(t)$ and $s_{IR}(t)$:

$$s_{IR}(t) = a(t) + v(t) + s_{IR0} - g(p(t))$$

$$s_R(t) = r_a{}^*a(t) + r_v{}^*v(t) + s_{R0} - g(p(t))$$

and further:

$$s_{IR}(t) = a(t) + v(t) + s_{IR0} - g(SP + h(a(t)))$$

$$s_R(t) = r_a{}^*a(t) + r_v{}^*v(t) + s_{R0} - g(SP + h(a(t)))$$

where g again describes the transfer function of cuff pressure on the finger. From the above formulae it can be seen that the plethysmographic measurement signals $s_R(t)$ and $s_{IR}(T)$ depend on a(t) via the response of the control loop g (SP + h(a(t))).

Properties of the filter

The problem of separating the two signals a(t) and v(t) lies in the fact that both signals share the same frequency band. If this were not the case separation could be effected by relatively simple frequency filters (high-pass, low-pass, band-pass or band-stop filters). A further problem is posed by the fast changes the venous signal may undergo. This suggests the preferential use of an "adaptive filter", i.e. a filter which can adapt its frequency characteristic to the given circumstances. It should be pointed out that such a filter in theory could also be built as a hardware device from conventional analog electronic elements. Preferably, however, this filter will be realized as a digital filter and implemented as software in a computer. The present invention does not discern between an analog filter and the digital version.

The present invention utilizes the fact that arterial blood at a certain wavelength has an absorption coefficient differing from that of venous blood. The separation process also must take into account the characteristic property of the vascular unloading technique, viz. that the signal derived from the transmitted or reflected light is minimized by the counter- pressure applied.

A reference signal n(t) is generated from the signals $s_R(t)$, $s_{IR}(t)$ and p(t), which has the same frequency characteristics as the venous signal v(t). Ideally $r_a$ is chosen for the determination of n(t):

$$n(t) = s_R(t) - r_a{}^*s_{IR}(t)$$

$$n(t) = r_a{}^*a(t) + r_v{}^*v(t) + s_{R0} - g(SP + h(a(t))) - r_a{}^*(a(t) + v(t) + s_{IR0} - g(SP + h(a(t))))$$

$$n(t) = r_v{}^*v(t) + s_{R0} - g(SP + h(a(t))) - r_a{}^*v(t) - r_a{}^*s_{IR0} + r_a{}^*g(SP + h(a(t)))$$

Suppressing mean values one has:

$$\Delta n(t)=v(t)*(r_v-r_a)+g(SP+h(a(t)))*(r_a-1)$$

$$\Delta n(t)=v(t)*(r_v-r_a)+g(SP+p(t))*(r_a-1)$$

Since $g^{-1}$ = h (the controller transfer function) and vice versa $h^{-1}$ = g , and since SP +$\Delta$p(t) is known, g(SP+$\Delta$p(t))*($r_a$-1) may be computed and subtracted and there remains:

$$\Delta n'(t)=v(t)*(r_v-r_a)+g(SP+\Delta p(t))*(r_a-1)-h^{-1}*(SP+\Delta p(t))*(r_a-1)$$

$$\Delta n'(t)=v(t)*(r_v-r_a)$$

$\Delta$n'(t) now has the same frequency properties as v(t). This signal may now be used to adjust an adaptive digital filter in such a way that it has the same frequency properties. The computation of such an "adaptive, autoregressive filter" in an other context has for instance been described in "Fortin J., Hagenbacher W., Gruellenberger R., Wach P., Skrabal F.: Real-time Monitor for Hemodynamic Beat-to-beat Parameters and Power Spectra Analysis of the Biosignals. Proceedings of the 20th Annual International Conference IEEE Engineering in Medicine and Biology Society, Vol 20, No 1, 360-3, 1998" or in "Schloegl A., Fortin J., Habenbacher W., Akay M.: Adaptive Mean and Trend Removal of Heart rate Variability using Kalman Filtering. Proceedings of the 23rd Annual International Conference of the IEEE Engineering in Medicine and Biology Society, Istanbul, 25-28 Oct. 2001, Paper #1383, ISBN 0-7803-7213-1.".

If one of the two original plethysmographic signals $s_R(t)$ or $s_{IR}(t)$ is filtered by this filter the arterial signal a(t) results, since it is known that in signal analysis there is no distinction between frequency properties and temporal changes (equality of the time domain and the frequency domain). $\Delta$n'(t) is computed continuously and determines or adapts the filter coefficients for one of the two signals $s_R(t)$ or $s_{IR}(t)$, and the resulting a(t) in turn serves as input signal for the controller.

Determination of the absomtion coefficients

To compute a(t), v(t), and n(t) $r_a$ and $r_v$ must be known. This is not the case as the oxygen saturation of the patient is not known initially. The trick used in this context is to obtain r by a series of trials. It is known that r is a function of oxygen saturation. The function Sp02 = f(r) has been found empirically. At r=1 one has for instance an oxygen saturation of 87% (to be exact, 86.69%). Furthermore oxygen saturation (venous and arterial) must lie in the physiological range, i.e. at the most between 30% and 100%. This gives a natural range of r-values of r=[2.46, 0.4]. A sufficiently accurate determination of Sp02 will be possible if measurement is exact to +/- 1%. Thus there will result e.g. J=71 possible r-values when Sp02 lies in [30% - 100%] or r =[2.46, 0.40].

A certain r is initially selected and the reference signal n(t) in the time domain or N(f) in the frequency domain is computed, which corresponds to the relevant filter transfer coefficient:

$$n(t)=s_R(t)-r*s_{IR}(t)$$

$$n(t)=r_a*a(t)+r_v*v(t)+s_{R0}-g(SP+\Delta p(t))-r*(a(t)+v(t)+s_{IR0}-g(SP+\Delta p(t)))$$

After means have been suppressed one has:

$$\Delta n(t)=(r_a-r)*a(t)+(r_v-r)*v(t)+(r-1)*g(SP+\Delta p(t))$$

If $(r-1)*h^{-1}(SP+p(t))$ is again subtracted from the reference signal one has:

$$\Delta n'(t)=(r_a-r)*a(t)+(r_v-r)*v(t)+(r-1)*g(SP+p(t))-(r-1)*h^{-1}(SP + p(t))$$

$$\Delta n'(t)=(r_a-r)*a(t)+(r_v-r)*v(t)$$

If the operation with $(r-1)*h^{-1}(SP+\Delta p(t))$ is not completely successful, because the physiological transfer function g is yet somewhat different from the controller transfer function h, a small residual part (factor c) of the $g(SP+\Delta p(t))$ signal will also remain, which vanishes only at $r = 1$:

$$\Delta n'(t)=(r_a-r)*a(t)+(r_v-r)*v(t)+c*(r-1)*g(SP+\Delta p(t))$$

It should be remembered that $\Delta n'(t)$ is measured from:

$$\Delta n'(t)=s_R(t)-r*s_{IR}(t)-mean(s_R(t)-r*s_{IR}(t))-(r-1)*h^{-1}(SP+\Delta p(t))$$

$$\Delta n'(t)=\Delta s_R(t)-r*\Delta s_{IR}(t)-(r-1)*h^{-1}(p(t))$$

Often it is easier to invert the frequency characteristic h of the controller, respectively to filter p(t) with the inverse frequency characteristic of the controller. In this case the reference signal would be obtained as:

$$\Delta n'(t)=\Delta s_R(t)-r*\Delta s_{IR}(t)-(r-1)*H^{-1}(p(t))$$

By letting r sequentially assume values from the range r => [30% - 100%] Sp02, one distinguishes the following four cases:

1) $r=r_a$ $\quad$ $\Delta n'(t)=(r_v-r_a)*v(t)+c*(r_a-1)*g(p(t))$

2) $r=r_v$ $\quad$ $\Delta n'(t)=(r_a-r_v)*a(t)+c*(r_v-1)*g(p(t))$

3) $r=1$ $\quad$ $\Delta n'(t)=(r_a-1)*a(t)+(r_v-1)*v(t)$

4) $r\neq r_a,r\neq r_v,r\neq 1$ $\quad$ $\Delta n'(t)=(r_a-r)*a(t)+(r_v-r)*v(t)+c*(r-1)*g(p(t))$

If filtering as described above is now carried out sequentially for all [i=1 to J] r-values, the respective output power P of the adaptive filter can be computed. It will be maximal in cases 1 - 3 above, in the fourth case, where r is not equal to one of the special values $r_a$, $r_v$ or 1, the output power is small. By plotting the output power for all J consecutive r-values or Sp02-values the correct values for $r_a$ and $r_v$ can be identified. $r_a$ or arterial saturation corresponds to the highest oxygen saturation present, or rather to the highest occurring local maximum of output power. At the point r=1 or SpO2=87% a local maximum occurs, which corresponds to the residual g(p(t)). The local maximum lying below these two r-values or Sp02-values corresponds to venous saturation. It is possible that the arterial saturation is precisely 87% and thus coincides with the local maximum. This can also be recognized by suitable logical queries. Furthermore, the maxima for venous saturation and g(p(f)) may be absent. The maximum for arterial saturation will always be present, however, and only this maximum is important for determining the correct reference signal and for SpO2-determination.

Once the arterial oxygen saturation and the corresponding r-value have been found, the correct filter for separating arterial from venous blood has also been determined. That filter which delivers the highest output power below the Sp02-value of 100%, or whose local maximum of output power has the highest oxygen saturation value, is the filter to select. It will separate a(t) and v(t) as computed from one of the original plethysmographic signals $S_R(t)$ or $S_{IR}(t)$.

Optimizing the controller

A further advantage of the present invention lies in the optimisation of the control mechanism. Here two values are of interest - the amplitude of a(t), which is minimized by the controller, and the output power at r=1. This corresponds to the degree of matching between the physiological transfer function g and the controller transfer function h.

For optimising a(t) the power of a(t) may be computed, which must be minimized by a suitable choice of h - or to be more precise - of the amplification of the controller. If the amplification of h is chosen too high the system starts to oscillate. In general control amplification is determined in the so-called "open loop phase". By measuring the power of a(t) the amplification may now also be optimised during continuous blood pressure measurement.

Again, measuring the output power of the filter at r=1 may be used for that. This output power normally corresponds to that of any other filter at r≠1. If the power is higher there, however, h ≠ g$^{-1}$. By adjusting h this may be compensated.

Speed Optimization

The values for $r_a$ or $r_v$ are determined from the output power of the J (adaptive) filters, and from $r_a$ and $r_v$ a(t) and v(t) are subsequently determined via the formulae given above. Since there will inevitably occur a certain time delay in the filters, this can cause problems for the control of pressure p(t). It would be of advantage, if especially a(t), which is needed as input variable for the control system, could be determined in optimal time. Since one may assume that $r_a$ and $r_v$, respectively the arterial and venous oxygen saturation, will not change during very short time intervals (e.g. in milliseconds), a variant of the present invention may be proposed. $r_a$ and $r_v$ are determined as described above from the set of J filters for the r values, taking the time required. Once $r_a$ and $r_v$ are given a(t) and v(t) may however be computed in real time from $S_R(t)$ and $s_{IR}(t)$ using the formulae already described above.

$$a(t) = \frac{\Delta s_R(t) - r'_v \cdot \Delta s_{IR}(t)}{r'_a - r'_v}$$

$$v(t) = \frac{\Delta s_R(t) - r'_a \cdot \Delta s_{IR}(t)}{r'_v - r'_a}$$

Optimization of the J filters, respectively the r-values

A further variant of the invention arises from the following consideration. According to the description above the J filters are plotted for instance over the range [30%-100%] of oxygen saturation at equal intervals of 1%. Over a certain region this is probably a too high resolution, while in the interesting region, where the output power for $r_a$, $r_v$ and 1 is located, a higher resolution might be desirable. The situation may be improved by weighting the intervals between successive r-values corresponding to Sp02-values in dependence of the output power. The higher the output power of the filter, the smaller the interval to the next filter and, vice versa, the smaller the output power the greater the interval. At the beginning of measurement, when the output power values are still unknown, an equidistant scale could be used, which in the course of measurement might be adjusted to provide better resolution.

**Abbreviations**

s(t)         plethysmographic measurement signal or volume signal
a(t)         arterial signal component of s(t) - favored signal
v(t)         venous signal component of s(t) - supplementing signal
$\Delta s(t)$         pulsatile component of the s(t)
$S_0$         mean value of s(t)
$S_{mean}$         mean value of s(t) as computed by the system
$S_R(t)$         Measurement or volume signal of red light
$S_{IR}(t)$         Measurement or volume signal of infrared light
p(t)         time-varying pressure signal - blood pressure
SP         set point of pressure
h or H         transfer function (time- vs. frequency domain)
g or G         transfer function (time- vs. frequency domain)
SpO2         oxygen saturation
aSp02         arterial oxygen saturation
vSp02         venous oxygen saturation
r         optical density ratio
$r_a$         optical density ratio for arterial blood
$r_v$         optical density ratio for venous blood
J         number of filters
n(t)         reference signal in the time domain
N(f)         reference signal or filter transfer function
$\Delta n'(t)$         pulsatile reference signal, with H suppressed

**Claims**

1. A device for the continuous, non-invasive measurement of the arterial blood pressure comprising:

   (a) a first radiation source (1) and at least one other radiation source (2) for generating a first and at least one other measurement radiation of defined, mutually differing wavelengths;

   (b) at least one detector (4) for generating a first measurement signal $s_1(t)$ from the first measurement radiation and at least one other measurement signal $s_N(t)$ from the at least one other measurement radiation of different wavelength, wherein the measurement radiations propagate wholly or partially along a propagation path and wherein at least a portion of this propagation path is located in a body part (3) traversed by arterial and venous blood flows, and wherein the first signal $s_1(t)$ has a first arterial signal component $a_1(t)$ and a first venous signal component $v_1(t)$ and wherein the at least one other signal $s_N(t)$ has at least one other arterial signal component $a_N(t)$ and at least one other venous signal component $v_N(t)$, and wherein arterial signal components $a_1(t)$ to $a_N(t)$ result from a time-varying arterial blood flow a(t) in the body part (3), and the venous signal components $v_1(t)$ to $v_N(t)$ result from a time-varying venous blood flow v(t) in the body part;

   (c) an air pressure generator (10), one or more valves (9), a manometer (11) and a cuff (12) for applying a time-varying pressure to the body part (3), wherein a pressure signal p(t) corresponding to an arterial blood pressure, is a function of the arterial blood flow a(t) in the body part (3) or a function of one or more of the signals $s_1(t)$ to $s_N(t)$ measured by the detector (4);

   (d) a reference signal generator (6), which has as inputs the signals $s_1(t)$ to $s_N(t)$ measured by the detector (4) and the pressure signal p(t), and which computes from these inputs a reference signal $\Delta n'(t)$, which is a function of the venous blood flow v(t) or of the venous signal components $v_1(t)$ to $v_N(t)$; and

   (e) a filter (7) receiving the reference signal $\Delta n'(t)$ as an input, where the frequency properties of the filter (7) essentially correlate with the reference signal $\Delta n'(t)$, and wherein the filter (7) essentially separates from at least in one of the signals $s_1(t)$ to $s_N(t)$ measured by the detector (4) the venous signal component $v_1(t)$ to $v_N(t)$ from the arterial signal component $a_1(t)$ to $a_N(t)$, wherein the arterial signal component is proportional to the arterial blood flow a(t).

2. The device according claim 1, wherein the filter (7) is an adaptive filter which can adapt its frequency characteristic during signal analysis by means of the reference signal.

3. The device according claim 1 or 2, wherein said device comprises means for deriving and displaying the arterial oxygen saturation aSp02 and/or the venous oxygen saturation vSp02 from the frequency properties obtained by measuring the blood pressure.

**EP 2 101 633 B1**

4. The device according to any of claims 1 to 3, wherein the first radiation source (1) generates red light of e.g. 660 nm and the at least one other radiation source (2) generates infrared light of e.g. 940 nm as measurement radiations.

5. A method for the continuous, non-invasive measurement of arterial blood pressure in a body part (3) with arterial and venous blood flow comprising:

(a) providing a first and at least one other measurement radiation of defined, mutually differing wavelengths;
(b) detecting a first measurement signal $s_1(t)$ from the first measurement radiation and at least one other measurement signal $s_N(t)$ from the at least one other measurement radiation of different wavelength, where the two measurement radiations propagate wholly or partially along the same propagation path and wherein part of this propagation path is located in the body part (3) in which arterial and venous blood flows, and wherein the first signal $s_1(t)$ has a first favored signal component $a_1(t)$ and a first supplementing signal component $v_1(t)$, and wherein the at least one other signal $s_N(t)$ has a favored signal component $a_N(t)$ and a supplementing signal component $v_N(t)$, and wherein the first and all other favored signal components $a_1(t)$ to $a_N(t)$ result from a time-varying arterial blood flow $a(t)$ in the body part (3) and the first and all other supplementing signal components $v_1(t)$ to $v_N(t)$ result from a time-varying venous blood flow $v(t)$ in the body part (3);
(c) applying a time-varying pressure to the body part (3), wherein a pressure signal $p(t)$ corresponding to the arterial blood pressure is a function of the arterial blood flow $a(t)$ in the body part or a function of one or more of the signals $s_1(t)$ to $s_N(t)$;
(d) computing a reference signal $\Delta n'(t)$ from the signals $s_1(t)$ to $s_N(t)$ and the pressure signal $p(t)$, which is a function of venous blood flow $v(t)$ or of the supplementing signal components $v_1(t)$ to $v_N(t)$; and
(e) separating the supplementing signal component $v_1(t)$ to $v_N(t)$ from the favored signal component $a_1(t)$ to $a_N(t)$ of the signals $s_1(t)$ to $s_N(t)$ measured by a detector (4) by means of a filter (7) receiving the reference signal $\Delta n'(t)$ as an input, wherein the frequency properties of the filter (7) essentially correlates with the reference signal $\Delta n'(t)$, and wherein the favored signal component $a_1(t)$ to $a_N(t)$ is proportional to the arterial blood flow $a(t)$.

6. The method according to claim 5, wherein the frequency properties of the filter (7) are adaptively modified during signal analysis by means of the reference signal.

7. The method according to claim 5 or 6, wherein from the frequency properties obtained by measuring the blood pressure, the arterial oxygen saturation aSp02 and/or the venous oxygen saturation vSp02, are derived and displayed.

8. The method according to any of claims 5 to 7, wherein red light is used as the first measurement radiation and infrared light is used as at least on other measurement radiation.


**Patentansprüche**

1. Vorrichtung für die kontinuierliche, nicht-invasive Messung des arteriellen Blutdrucks, umfassend:

(a) eine erste Strahlungsquelle (1) und wenigstens eine weitere Strahlungsquelle (2) zur Erzeugung einer ersten und wenigstens einer weiteren Messstrahlung von definierten, sich gegenseitig unterscheidenden Wellenlängen;
(b) wenigstens einen Detektor (4) zur Erzeugung eines ersten Messsignals $s_1(t)$ aus der ersten Messstrahlung und wenigstens eines weiteren Messsignals $s_N(t)$ aus der wenigstens einen weiteren Messstrahlung von unterschiedlicher Wellenlänge, wobei sich die Messstrahlungen gänzlich oder teilweise entlang einem Ausbreitungsweg fortpflanzen und wobei sich wenigstens ein Teil dieses Ausbreitungsweges in einem Körperteil (3) befindet, das von arteriellen und venösen Blutströmen durchflossen wird, und wobei das erste Signal $s_1(t)$ eine erste arterielle Signalkomponente $a_1(t)$ und eine erste venöse Signalkomponente $v_1(t)$ aufweist und wobei das wenigstens eine weitere Signal $s_N(t)$ wenigstens eine weitere arterielle Signalkomponente $a_N(t)$ und wenigstens eine weitere venöse Signalkomponente $v_N(t)$ aufweist und wobei die arteriellen Signalkomponenten $a_1(t)$ bis $a_N(t)$ von einem zeitlich veränderlichen arteriellen Blutstrom $a(t)$ in dem Körperteil (3) herrühren und die venösen Signalkomponenten $v_1(t)$ bis $v_N(t)$ von einem zeitlich veränderlichen venösen Blutstrom $v(t)$ in dem Körperteil herrühren;
(c) einen Luftdruckgenerator (10), ein oder mehrere Ventile (9), ein Manometer (11) und eine Manschette (12) zum Anlegen eines zeitlich veränderlichen Drucks an den Körperteil (3), wobei ein Drucksignal $p(t)$, das einem arteriellen Blutdruck entspricht, eine Funktion des arteriellen Blutstroms $a(t)$ in dem Körperteil (3) oder eine Funktion eines oder mehrerer der von dem Detektor (4) gemessenen Signale $s_1(t)$ bis $s_N(t)$ ist;

(d) einen Referenzsignalgenerator (6), der als Eingangsgrößen die von dem Detektor (4) gemessenen Signale $s_1(t)$ bis $s_N(t)$ und das Drucksignal $p(t)$ hat und der aus diesen Eingangsgrößen ein Referenzsignal $\Delta n'(t)$ berechnet, das eine Funktion des venösen Blutstroms $v(t)$ oder der venösen Signalkomponenten $v_1(t)$ bis $v_N(t)$ ist; und

(e) ein Filter (7), das als Eingangsgröße das Referenzsignal $\Delta n'(t)$ empfängt, wobei die Frequenzeigenschaften des Filters (7) im Wesentlichen mit dem Referenzsignal $\Delta n'(t)$ korrelieren und wobei das Filter (7) im Wesentlichen bei wenigstens einem der von dem Detektor (4) gemessenen Signale $s_1(t)$ bis $s_N(t)$ die venöse Signalkomponente $v_1(t)$ bis $v_N(t)$ von der arteriellen Signalkomponente $a_1(t)$ bis $a_N(t)$ trennt, wobei die arterielle Signalkomponente proportional zum arteriellen Blutstrom $a(t)$ ist.

2. Vorrichtung nach Anspruch 1, wobei das Filter (7) ein adaptives Filter ist, das seine Frequenzeigenschaften während der Signalanalyse mittels des Referenzsignals anpassen kann.

3. Vorrichtung nach Anspruch 1 oder 2, wobei diese Vorrichtung Mittel zur Ableitung und Anzeige der arteriellen Sauerstoffsättigung aSp02 und/oder der venösen Sauerstoffsättigung vSp02 aus den durch Blutdruckmessung erhaltenen Frequenzeigenschaften umfasst.

4. Vorrichtung nach einem der Ansprüche 1 bis 3, wobei die erste Strahlungsquelle (1) als Messstrahlung rotes Licht von z.B. 660 nm erzeugt und die wenigstens eine weitere Strahlungsquelle (2) als Messstrahlung Infrarotlicht von z.B. 940 nm erzeugt.

5. Verfahren zur kontinuierlichen, nicht-invasiven Messung des arteriellen Blutdrucks in einem Körperteil (3) mit arteriellem und venösem Blutstrom, umfassend:

(a) Bereitstellen einer ersten und wenigstens einer weiteren Messstrahlung von definierten, sich gegenseitig unterscheidenden Wellenlängen;

(b) Erfassung eines ersten Messsignals $s_1(t)$ aus der ersten Messstrahlung und wenigstens eines weiteren Messsignals $s_N(t)$ aus der wenigstens einen weiteren Messstrahlung von unterschiedlicher Wellenlänge, wobei sich die zwei Messstrahlungen gänzlich oder teilweise entlang demselben Ausbreitungsweg fortpflanzen und wobei sich ein Teil dieses Ausbreitungsweges in dem Körperteil (3) befindet, in dem arterielles und venöses Blut strömt, und wobei das erste Signal $s_1(t)$ eine erste bevorzugte Signalkomponente $a_1(t)$ und eine erste ergänzende Signalkomponente $v_1(t)$ aufweist und wobei das wenigstens eine weitere Signal $s_N(t)$ eine bevorzugte Signalkomponente $a_N(t)$ und eine ergänzende Signalkomponente $v_N(t)$ aufweist und wobei die erste und alle weiteren bevorzugten Signalkomponenten $a_1(t)$ bis $a_N(t)$ von einem zeitlich veränderlichen arteriellen Blutstrom $a(t)$ in dem Körperteil (3) herrühren und die erste und alle weiteren ergänzenden Signalkomponenten $v_1(t)$ bis $v_N(t)$ von einem zeitlich veränderlichen venösen Blutstrom $v(t)$ in dem Körperteil (3) herrühren;

(c) Anlegen eines zeitlich veränderlichen Drucks an den Körperteil (3), wobei ein Drucksignal $p(t)$, das dem arteriellen Blutdruck entspricht, eine Funktion des arteriellen Blutstroms $a(t)$ in dem Körperteil (3) oder eine Funktion eines oder mehrerer der Signale $s_1(t)$ bis $s_N(t)$ ist;

(d) Berechnen eines Referenzsignals $\Delta n'(t)$ aus den Signalen $s_1(t)$ bis $s_N(t)$ und dem Drucksignal $p(t)$, das eine Funktion des venösen Blutstroms $v(t)$ oder der ergänzenden Signalkomponenten $v_1(t)$ bis $v_N(t)$ ist; und

(e) Trennen der ergänzenden Signalkomponente $v_1(t)$ bis $v_N(t)$ von der bevorzugten Signalkomponente $a_1(t)$ bis $a_N(t)$ der von einem Detektor (4) gemessenen Signale $s_1(t)$ bis $s_N(t)$ mittels eines Filters (7), das als Eingangsgröße das Referenzsignal $\Delta n'(t)$ empfängt, wobei die Frequenzeigenschaften des Filters (7) im Wesentlichen mit dem Referenzsignal $\Delta n'(t)$ korrelieren und wobei die bevorzugte Signalkomponente $a_1(t)$ bis $a_N(t)$ proportional zum arteriellen Blutstrom $a(t)$ ist.

6. Verfahren nach Anspruch 5, wobei die Frequenzeigenschaften des Filters (7) während der Signalanalyse mittels des Referenzsignals adaptiv verändert werden.

7. Verfahren nach Anspruch 5 oder 6, wobei aus den durch Blutdruckmessung erhaltenen Frequenzeigenschaften die arterielle Sauerstoffsättigung aSp02 und/oder die venöse Sauerstoffsättigung vSp02 abgeleitet und angezeigt werden.

8. Verfahren nach einem der Ansprüche 5 bis 7, wobei rotes Licht als erste Messstrahlung verwendet wird und Infrarotlicht als wenigstens eine weitere Messstrahlung verwendet wird.

**Revendications**

1. Dispositif pour la mesure continue non invasive de la pression sanguine artérielle comprenant :

(a) une première source de rayonnement (1) et au moins une autre source de rayonnement (2) pour créer un premier et au moins un autre rayonnement de mesure ayant des longueurs d'ondes définies différentes entre elles,

(b) au moins un détecteur (4) pour créer un premier signal de mesure $s_1(t)$ à partir du premier rayonnement de mesure et au moins un autre signal de mesure $s_N(t)$ à partir du ou des autres rayonnement(s) de mesure de longueur(s) d'ondes différente(s), étant précisé que les rayonnements de mesure se propagent en totalité ou partiellement le long d'un parcours de propagation, dont au moins une partie est localisée dans une partie (3) du corps traversé par des flux sanguins veineux et artériels, que le premier signal $s_1(t)$ a une première composante de signal artérielle a $s_1(t)$ et une première composante de signal veineuse $v_1(t)$, tandis que le ou les autres signal(ux) $s_N(t)$, a(ont) au moins une autre composante de signal artérielle $a_N(t)$ et au moins une autre composante de signal veineuse $v_N(t)$, et que les composantes de signal artérielles a(t) à $a_N(t)$ résultent d'un flux sanguin artériel variable dans le temps a(t) dans la partie du corps (3) tandis que les composantes de signal veineuses $v_1(t)$ à $v_N(t)$ résultent d'un flux sanguin veineux variable dans le temps v(t) dans la partie du corps,

(c) un générateur de pression d'air (10) au moins une soupape (9) un manomètre (11) et un brassard (12) pour appliquer une pression variable dans le temps à la partie du corps (3) étant précisé que le signal de pression p(t) correspondant à la pression sanguine artérielle est une fonction du flux sanguin artériel a(t) dans la partie du corps (3) ou une fonction d'au moins l'un des signaux Sn(t) à $s_N(t)$ mesurés par le détecteur (4),

(d) un générateur de signal de référence (6) recevant en entrées les signaux $s_1(t)$ à $s_N(t)$ mesurés par le détecteur (4) et le signal de pression p(t) et calculant à partir de ces entrées, un signal de référence Δn'(t) qui est une fonction du flux sanguin veineux v(t) ou des composantes de signal veineuses $v_1(t)$ à $v_N(t)$, et

(e) un filtre (7) recevant le signal de référence Δn'(t) en entrée, étant précisé que les propriétés de fréquence du filtre (7) correspondent essentiellement au signal de référence Δn'(t), et que le filtre (7) sépare essentiellement d'au moins l'un des signaux $s_1(t)$ à $s_N(t)$ mesuré par le détecteur (4), la composante de signal veineuse $v_1(t)$ à $v_N(t)$ de la composante de signal artérielle $a_1(t)$ à $a_N(t)$, la composante de signal artérielle étant proportionnelle au flux sanguin artériel a(t).

2. Dispositif conforme à la revendication 1,
dans lequel
le filtre (7) est un filtre adaptatif susceptible d'adapter sa caractéristique de fréquence au cours de l'analyse du signal au moyen du signal de référence.

3. Dispositif conforme à la revendication 1 ou 2,
comportant des moyens pour dériver et afficher la saturation artérielle en oxygène aSp02 et/ou la saturation veineuse en oxygène vSp02 à partir des propriétés de fréquence obtenues en mesurant la pression sanguine.

4. Dispositif conforme à l'une des revendications 1 à 3,
dans lequel
la première source de rayonnement (1) émet une lumière rouge d'approximativement 660 nm, et la ou les autre(s) source(s) de rayonnement (2) émet(tent) de la lumière infrarouge d'approximativement 940 nm en tant que rayonnements de mesure.

5. Procédé de mesure continue non invasive de la pression sanguine artérielle dans une partie du corps (3) ayant un flux sanguin veineux et artériel comportant les étapes consistant à :

(a) se procurer un premier et au moins un autre rayonnement de mesure ayant des longueurs d'ondes définies différentes,

(b) détecter un premier signal de mesure $s_1(t)$ à partir du premier rayonnement de mesure et au moins un autre signal de mesure $s_N(t)$ à partir du ou des autre(s) rayonnement(s) de mesure de longueur(s) d'ondes différente (s), étant précisé que les deux rayonnements de mesure se propagent en totalité ou partiellement le long du même parcours de propagation, dont une partie est située dans la partie du corps (3) dans laquelle s'écoule le flux sanguin veineux et artériel, que le premier signal $s_1(t)$ comporte une première composante de signal privilégiée $a_1(t)$ et une première composante de signal supplémentaire $v_1(t)$, tandis que le ou les autre(s) signal(ux) $s_N(t)$ comporte(nt) une composante de signal privilégiée $a_N(t)$ et une composante de signal supplémentaire $v_N(t)$, et que la première et toutes les autres composantes de signal privilégiées $a_1(t)$ à $a_N(t)$ résultent d'un flux sanguin artériel variable dans le temps a(t) dans la partie du corps (3) tandis que la première et toutes les autres composantes de signal supplémentaires $v_1(t)$ à $v_N(t)$, résultent d'un flux sanguin veineux variable dans le temps v(t) dans la partie du corps (3),

(c) appliquer une pression variable dans le temps à la partie du corps (3), étant précisé que le signal de pression p(t) correspondant à la pression sanguine artérielle est une fonction du flux sanguin artériel a(t) dans la partie du corps ou une fonction d'au moins l'un des signaux $s_1(t)$ à $s_N(t)$,

(d) calculer un signal de référence $\Delta n'(t)$ à partir des signaux $s_1(t)$ à $s_N(t)$ et du signal de pression p(t) qui est une fonction du flux sanguin veineux v(t) ou des composantes de signal supplémentaires $v_1(t)$ à $v_N(t)$, et

(e) séparer les composantes de signal supplémentaires $v_1(t)$ à $v_N(t)$ des composantes de signal privilégiées $a_1(t)$ à $a_N(t)$ des signaux $s_1(t)$ à $s_N(t)$ mesurés par un détecteur (4) au moyen d'un filtre (7) recevant le signal de référence $\Delta n'(t)$ en entrée, étant précisé que les propriétés de fréquence du filtre (7) correspondent essentiellement au signal de référence $\Delta n'(t)$, et que la composante de signal privilégiée $a_1(t)$ à $a_N(t)$ est proportionnelle au flux sanguin artériel a(t).

6. Procédé conforme à la revendication 5,
   selon lequel
   les propriétés de fréquence du filtre (7) sont modifiées de façon adaptative au cours de l'analyse du signal au moyen du signal de référence.

7. Procédé conforme à la revendication 5 ou 6,
   selon lequel
   les propriétés de fréquence obtenues en mesurant la pression sanguine, la saturation artérielle en oxygène aSp02 et/ou la saturation veineuse en oxygène vSp02, sont dérivées et affichées.

8. Procédé conforme à l'une des revendications 5 à 7,
   selon lequel
   de la lumière rouge est utilisée en tant que premier rayonnement de mesure tandis que de la lumière infrarouge est utilisée en tant qu'autre(s) rayonnement(s) de mesure.

Fig. 1

Fig. 2

Fig. 3

**Optical Density Ratio r**

Fig. 4

**Filter Output Power**

Fig. 5a

**Filter Output Power**

Fig. 5b

**Filter Output Power**

Fig. 5c

**Weighted Output Power**

Fig. 6a

**Weighted Output Power**

Fig. 6b

**Weighted Output Power**

Fig. 6c

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0537383 A1
- US 4510940 A
- US 4539997 A
- US 4597393 A
- WO 0059369 A2
- WO 04086963 A2
- WO 05037097 A1
- US 4653498 A
- US 5025791 A
- US 4802486 A
- US 5078136 A
- US 5337744 A
- US 6845256 A
- US 20050256386 A

- US 5769785 A
- US 6036642 A
- US 6157850 A
- US 6206830 A
- US 6263222 A
- WO 9215955 A
- EP 0574509 B1
- DE 69229994
- WO 9612435 A2
- JP 6063024 A, Igarashi
- JP 2305555 A, Yamakoshi
- US 5485838 A, Ukawa
- US 5111817 A, Clark
- US 4927264 A, Shiga

**Non-patent literature cited in the description**

- Methodik und Ergebnisse fort-laufender Blutdruck-schreibung am Menschen. **Wagner R.** Leipzig. Georg Thieme Verlag, 1942
- **Wagner R. et al.** Vereinfachtes Verfahren zur fortlaufenden Aufschrift des Blutdrucks beim Menschen. *Zschr. Biol.,* 1960, vol. 112
- **A.F.M. Smith ; M. West.** Monitoring Renal Transplants: An Application for the Multiprocess Kalman Filter. *Biometrics,* 1983, vol. 39, 867-878
- **K. Gordon.** The Multi State Kalman Filter in Medical Monitoring. *Computer Methods and Programs in Biomedicine,* 1986, vol. 23, 147-154

- **Fortin J. ; Hagenbacher W. ; Gruellenberger R. ; Wach P. ; Skrabal F.** Real-time Monitor for Hemodynamic Beat-to-beat Parameters and Power Spectra Analysis of the Biosignals. *Proceedings of the 20th Annual International Conference IEEE Engineering in Medicine and Biology Society,* 1998, vol. 20 (1), 360-3
- **Schloegl A. ; Fortin J. ; Habenbacher W. ; Akay M.** Adaptive Mean and Trend Removal of Heart rate Variability using Kalman Filtering. *Proceedings of the 23rd Annual International Conference of the IEEE Engineering in Medicine and Biology Society,* ISBN 0-7803-7213-1